# EUROPEAN PATENT APPLICATION

(11) **EP 2 510 969 A1**
(43) Date of publication of application: **17.10.2012**
(21) Application number: 11162140.5
(22) Date of filing: 12.04.2011
(51) Int. Cl.: A61M 16/12, A61G 12/00, F17D 1/02

(54) **System and method for supplying a medical gas in a medical facility**

(71) Applicant: Linde AG, 80331 München (DE)
(72) Inventor: Sage, Phillippe, 81310, Lisle sur Tarn (FR)
(74) Representative: Dörries, Hans Ulrich

(57) **Abstract**

The present invention pertains to a system for distributing a medical gas in a medical facility, preferably in a hospital, the system comprising at least one gas blender (3) for gas blending medical air and medical oxygen in a predetermined gas blending ratio, and at least one supply interface situated in a patient room (III), preferably a wall outlet (32), for supplying the gas blend to a device for treating a patient.

## Description

### Technical Field

The present invention pertains to a system and a method for supplying a medical gas in a medical facility, preferably in a hospital, a clinic or a rehabilitation center.

### Technical Background

In medical facilities such as hospitals, clinics or rehabilitation centers, certain medical gases are frequently supplied in a standardized manner to the respective treatment rooms, patient rooms or operating rooms. The gases are typically distributed from a centralized gas supply to the respective rooms by means of a pipeline system running throughout the medical facility, or at least throughout a unit of the medical facility. In the respective rooms, the respective gases are then supplied by means of suitable wall outlets from which a user such as a medical practitioner or a caregiver can easily take the gas by connecting a suitable device to the wall outlet. The device is then used to treat patients by administering the gas to the patient.

The wall outlets are usually provided in the vicinity of a patient bed or in the vicinity of the respective operation tables such that the desired gas can be supplied to the respective devices for treatment of the patients. In a typical hospital, gas wall outlets are available for medical oxygen, medical nitrous oxide, medical air and vacuum.

The vacuum wall outlet serves all applications where suction devices are used in connection with the respective treatments. Medical oxygen is supplied directly to a patient, whereas it is diluted with ambient air in most cases. Medical air is typically used for respiratory treatments and/or to feed respiratory machines used in the treatment of patients. Examples of patients which are dependent on a reliable supply of high quality air are neonates and adult patients suffering from respiratory conditions. Nitrous oxide is used for anesthetic and analgesic procedures.

Pure medical oxygen contains not less than 99.0% by volume of O₂ and the remaining gases are pharmaceutically neutral. Medical oxygen is fed into the pipeline system from a centralized supply, typically from a tank of liquid oxygen or from oxygen cylinders or oxygen cylinder bundles which are shipped to the hospital via vehicles. In an alternative setting a local oxygen generator could be used, for example in the form of a pressure swing adsorption means which is set to produce a gas with an oxygen content of about 93%.

Conventionally, medical air is typically fed into the hospital pipeline system either from air compressors in combination with advanced air filtering and air cleaning techniques such that ambient air is cleaned and filtered to produce the medical air on site, or from a centralized supply system, typically from a tank of liquid air or from cylinders or cylinder bundles which are shipped to the respective hospital via vehicles.

Medical nitrous oxide is fed into the hospital pipeline system typically from a centralized supply, typically in the form of a tank containing liquid nitrous oxide, or from cylinders or cylinder bundles which are shipped to the respective hospital via vehicles.

Vacuum is produced by means of a vacuum compressor, usually also in a centralized fashion.

For the treatment of patients with oxygen, different means are known to supply the patient with the gas. For example, a nasal cannula may be used which is situated below the nostrils of a patient and which provides a constant flow of oxygen directed towards the nostrils of the patient. Accordingly, the patient inhales a mixture of ambient air and medical oxygen wherein, however, the actual mixing ratio cannot exactly be set. As a constant flow of oxygen is supplied, oxygen is wasted at least when the patient exhales.

Another form of treating patients with oxygen is via a nasal catheter where a catheter is introduced through the nose and the catheter tip is placed behind the uvula such that oxygen is constantly supplied to the patient. Another form of treatment is with a tracheal catheter, which is surgically inserted into the trachea. Both methods of treatment require that the gas administered to the patient has an oxygen content of less than 100% in order to avoid damages of the lungs. Accordingly, medical oxygen taken from the wall outlet of the hospital pipeline system is mixed with ambient air before it is administered to the patient.

Other forms of treatments include the administration of the oxygen/ambient air mix by means of a mask covering the patient nose and mouth or through respiratory machines.

Whereas in intensive care applications, emergency rooms and operation rooms it might be necessary to have available pure medical oxygen, this is not the case in non-acute units of medical facilities where medical oxygen is always diluted by ambient air before it is administered to a patient. The oxygen content of the gases actually administered to these patients is typically below 70% and more typically below 50%.

### Summary

Accordingly, it is an objective of the present invention to provide a system and method for supplying medical gas in a medical facility which increases the efficiency of oxygen usage.

According to the present disclosure, a system for supplying a medical gas in a medical facility, preferably a hospital, is suggested with the features of claim 1.

The system comprises at least one gas blender for blending medical air and medical oxygen in a predetermined blending ratio. At least one supply interface is situated in a patient room, preferably in the form of a wall outlet, for supplying the gas blend to a device for treating the patient.

Because the oxygen content in the gas blend is lower than in pure medical oxygen, considerable savings in oxygen usage can be achieved by means of the system. For example, when administering the gas blend via a nasal cannula, less oxygen is wasted when the patient is exhaling because the relative flow of oxygen per unit of time is reduced. Furthermore, as a gas blend of oxygen and medical air is administered to the patient, the risk of inhaling contaminated ambient air which may be loaded with germs or pathogens, in particular in the context of a hospital, can be reduced or even avoided. Accordingly, the system not only has the advantage of increasing the efficiency of oxygen usage but, at the same time, hygienic conditions for the patients can be improved.

The increase in efficiency also stems from an improved use of medical oxygen according to the respective clinical protocols for the respective patient conditions. In acute care, patients may need a pure medical oxygen supply. Chronic and/or stable patient conditions require lower oxygen concentrations to be administered. The actual oxygen concentrations are to be defined by clinical prescription. For example, in a hospital specializing in long-term patients which, for example, require a maximum of 60% medical oxygen concentration in the gas blend, the savings in medical oxygen would be up to 60%: 21% oxygen content are already present in the medical air such that it is to be topped-up by roughly 40% medical oxygen only to achieve the desired gas blend.

As another advantage, the patient safety is further improved because it is no longer possible to accidentally administer a gas containing 100% oxygen to a patient because a pure oxygen supply is not present in the patient room any more. If administered in an uncontrolled manner over a long time, a gas containing 100% oxygen may lead to damages of the lungs. Scientists of the Vanderbilt University Medical Center reported that high oxygen concentrations may produce free radicals which consequently lead to hyperoxia-induced lung injuries.

Furthermore, the amount of oxygen which is administered to the patient can be more easily traced and controlled according to a medical prescription compared with the current situation in which medical oxygen is diluted by an arbitrary amount of ambient air. For example, currently the use of a nasal cannula would not give the medical practitioner any exact idea of how much oxygen is actually administered to the patient. To the contrary, even if a constant flow of oxygen were adjusted, the oxygen content inhaled varies from patient to patient, depending on the actual positioning of the nasal cannula as well as the relative dimensions of the cannula and the nostrils of the respective patient. The present system may lead to higher precision of the administration of the gas blend because defined gas blend could be administered to the patient directly.

In addition, with the system according to the present teachings, pharmaceutical grade gas can be administered to the patients only and the administration of ambient air can be avoided under certain circumstances. In fact, in the system according to the present disclosure two pharmaceutical grade gases are mixed and administered to the patient such that contaminations can be avoided and the dilution of the gas blend by means of ambient air can be avoided or at least reduced.

A device for treating a patient is understood to comprise any device which is intended to transport the gas blend from the supply interface to the patient in order to administer the gas blend to the patient. Using such a device for treating the patient is indispensable in the present system because the supply interface is located in a spaced apart manner from the head of the patient. Accordingly, at least the provision of a tube would be necessary to transport the gas blend from the supply interface to the patient in order to administer the gas blend to the patient. Typically such a device for treating a patient would be present in the form of a nasal cannula, a catheter, a mask or any other suitable device for transporting the gas blend from the supply interface to a patient.

Preferably, the system further comprises a supply of medical oxygen, a supply of medical air and pipelines for connecting the supply of medical air and the supply of medical oxygen to the gas blender. Furthermore, the system preferably includes at least one pipeline for connecting the gas blender to the supply interface. By means of this layout, the system according to the present disclosure can be applied to conventional hospital gas pipeline systems without the need for major reconstruction of the existing pipelines.

Preferably, the gas blender is set to provide an oxygen content in the gas blend of between 30% by volume to 96% by volume, preferably between 40% by volume to 80% by volume, more preferred between 45% by volume to 70% by volume, most preferred of 50% by volume. With these gas blending ratios it becomes possible to reduce the need for additional dilution of the gas blend by adding ambient air before it is actually administered to a patient. In other words, it is feasible to set a predetermined gas blending ratio and to administer the resulting gas blend directly to a patient. By doing so, the devices for administering the gas blend to the patient can be greatly simplified because a vent for ambient air can be omitted and the gas blend administered to the patient is free of any contamination that is typically associated with the addition of ambient air.

In a preferred embodiment, the system further comprises, in addition to the supply interface for the gas blend, at least one supply interface for medical air and at least one supply interface for vacuum. In this preferred embodiment, patient rooms without any supply interface for pure medical oxygen are provided. In other words, only three types of gas wall outlets are present in these patient rooms, namely one for the gas blend, one for medical air and one for vacuum.

In a hospital, it may be preferable to have a separate gas blender at every ward gas entry such that the gas blending ratios can be adjusted independently for every individual ward. The ward gas entry is not necessarily identical, in a spatial sense, with the ward entry through which persons enter into the respective ward. In particular, the ward gas entry is defined by the location at which a ward gas pipeline system is fed with the respective gases and may well be situated in a centralized area of the medical facility, spaced apart from the patient rooms of the actual ward. The gas blend is preferably supplied from the ward entry to the ward at a pressure of between 2 and 6 bar gauge (barg), more preferred at between 3 and 5 bar gauge (barg), most preferred at about 4 bar gauge (barg).

In a preferred embodiment, at least two gas blenders are provided which are set to supply blended gas with blending ratios distinct from one another, wherein preferably at least one gas blender is located at each ward gas entry of at least two different wards in order to supply blended gases having blending ratios which are distinct from one another to the different wards.

By means of the provision of the gas blender at the ward gas entry the gas blending ratio can be adjusted to the actual needs of the conditions of the patients treated in the specific ward.

The provision of a separate gas blender for every hospital ward also has the advantage that different areas of the hospital can be used flexibly for different purposes, without the need to change the layout of the pipeline system. In fact, the pipeline system which is already in place in a medical facility can be easily used for the system suggested in the present application. To this end, the gas blender is conveniently placed at the position in which the gas is fed into the pipelines which are intended to supply the respective areas of the medical facility. The gas blend produced in the gas blender is then fed into the pipeline which was formerly used for supplying pure medical oxygen.

In another preferred embodiment, a single gas blender is provided, preferably in a centralized location or outside of the hospital, for supplying specific sections or the entire hospital with the gas blend in a predetermined gas blending ratio, wherein the gas blend is preferably supplied at a pressure of between 5 and 15 bar gauge (barg), more preferably between 7 and 13 bar gauge (barg), even more preferred between 8 and 11 bar gauge (barg), most preferred at 10 bar gauge (barg) to the sections or the entire hospital. The provision of a single gas blender may improve the efficiency of the system because it reduces the number of appliances needed.

In order to avoid confusion of the users, in particular of the medical personnel such as doctors, medical practitioners and/or nurses, at the respective wall outlets, it is contemplated using a specifically shaped wall connector for connecting a device for treating a patient to the supply interface supplying the gas blend and/or to have specifically marked wall connectors for informing the users of the gas mixture which is actually delivered through the respective wall connectors. The shape of the wall connector may preferably be different from the wall connector that is typically used for the pure medical oxygen supply and/or clearly marked to unambiguously inform the care givers and users.

By means of the provision of gas blenders in every hospital ward or for a cluster of hospital wards, the supply logistics of the oxygen for the entire medical facility does not need to be changed. In particular, in the specific areas in which a pure medical oxygen supply is required, such as in emergency rooms or operating rooms, a pure medical oxygen supply can still be realized by means of appropriate mobile or fixed cylinders or cylinder bundles. Accordingly, the overall efficiency of the supply system for supplying gas to patients in a hospital can be significantly improved because the oxygen content of the medical gas supplied to the respective patients can be adjusted to the respective requirements in the different sections of the hospital.

As an advantageous side effect, the corrosion of the pipeline system in the areas in which the gas blend is supplied is reduced because of the reduced oxygen content of the gas blend.

Preferably, the gas blender is situated in a room or area different from the room where the respective supply interface is situated.

The medical oxygen may be supplied by means of a liquid oxygen tank, oxygen cylinders, by means of pressure swing adsorption technology (PSA), or by vacuum pressure swing adsorption technology (VPSA), or any other means known in the art to provide and/or produce medical grade oxygen in a medical facility. The pressure swing adsorption technology is well known in the art and is used to separate some gas species from a mixture of gases under pressure according to the respective gases' molecular characteristics.

Medical air is preferably provided by means of either air compressors with advanced air filtering and air cleaning techniques such that ambient air is cleaned and filtered to produce the medical air on site. The medical air may also be provided by means of a centralized supply system such as a tank of liquid medical air, or by means of cylinders or bundles of cylinders which are shipped to the hospital. Other means known in the art for providing medical grade air may also be used.

The supply interface for the gas blend preferably is provided in the form of a wall outlet and/or a wall socket which is formed for receiving a plug and/or tube of a device for treating a patient. Preferably the supply interface is a self-closing interface which only opens once a correspondingly shaped plug is inserted into the respective socket. Preferably, the supply interface is marked and/or shaped individually to inform users of the blending ratio supplied via the respective supply interface.

Preferably, in a patient room in a hospital, gas wall outlets for medical air, vacuum and the gas blend are provided only. In other words, a gas wall outlet for pure medical oxygen is not present.

Preferably, in different areas or different units of the hospital, different gas blending ratios for the medical air and medical oxygen may be set.

In the system, it is furthermore preferred that three different pipelines are used to supply a patient room with medical gases, namely a first pipeline for supplying medical air, a second pipeline for supplying vacuum and a third pipeline for supplying the gas blend. In this specific embodiment, a fourth pipeline for supplying pure medical oxygen is explicitly not provided for certain patient rooms.

Preferably, at least two gas blenders are present in the medical facility which are situated at two ward gas entries in order to provide different gas blending ratios of the gas blend to the two different medical wards or areas in the hospital. The gas blend is preferably supplied at a pressure of between 2 and 6 bar gauge (barg), preferably at between 3 and 5 bar gauge (barg), most preferred at about 4 bar gauge (barg) throughout the respective ward.

In an alternative, a single gas blender may be present centrally, supplying specific sections or the entire hospital with the gas blend in a predetermined gas blending ratio, wherein the gas blend is preferably supplied at a pressure of between 5 and 15 bar gauge (barg), preferably between 7 and 13 bar gauge (barg), more preferred between 8 and 11 bar gauge (barg), most preferred at 10 bar gauge (barg).

Preferably, an emergency valve and/or an emergency bypass is present at the gas blender, which provides an override of the gas blender such that, in an emergency, the supply can be switched immediately to pure medical oxygen, should this be required.

It is an advantage of the present system that no substantial modifications of the hospital pipeline system are needed because the gas blend of oxygen and medical air can be supplied via the regular pure medical oxygen pipeline.

The respective supply interfaces or supply outlets may be marked according to the oxygen concentration which is supplied through them.

The system may further comprise a supply of nitrous oxide and/or a supply of any other medical gas, and at least one further supply interface, preferably a wall outlet, in a patient room for supplying the nitrous oxide and/or the medical gas to a device for treating a patient. Nitrous oxide may be used for treating patients in analgesic or anesthetic procedures. The supply of the nitrous oxide via the wall outlet increases the efficiency in supplying the gas to the respective operating theaters or other rooms where anaesthesia/analgesia are provided to patients.

Preferably, the system further includes a blender for blending the nitrous oxide and/or the medical gas with medical oxygen and/or with medical air in a predetermined blending ratio, wherein the gas blend is supplied to a device for treating a patient via the supply interface, preferably the wall outlet, in the patient room. By this measure a ready-to-administer blend can be supplied via the hospital pipeline system and the risk of administering an hypoxic gas to a patient can be significantly reduced.

In order to easily and efficiently control the system, preferably a control system for remotely controlling the at least one gas blender is present. The control system may include means for measuring the blending ratio in the blended gas flow and for communicating the measured blending ratio to the control system.

The above mentioned objective is also solved by means of a method for supplying a medical gas in a medical facility with the steps of blending medical air and medical oxygen in a predetermined blending ratio by means of at least one gas blender and supplying the gas blend to a device for treating a patient by means of at least one supply interface situated in a patient room, preferably a wall outlet.

### Brief Description of the Drawings

In the following, preferred embodiments of the present disclosure will be described with respect to the following Figures:
- Figure 1: is a schematic view of a system according to the present disclosure;
- Figure 2: is a schematic view of another system according to the present disclosure;
- Figure 3: is a schematic view of a system according to the present disclosure including more than one blender;
- Figure 4: is a schematic view of a system according to the present disclosure, further including a nitrous oxide source.

### Detailed Description of the preferred Embodiments

In the following, a detailed description of the preferred embodiments will be given along the Figures. Similar or identical elements will be denoted by the same reference numerals and repeated description thereof may be omitted to reduce redundancy.

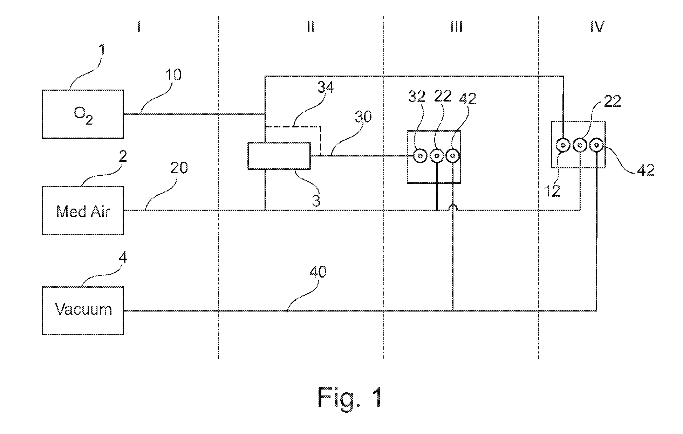

Figure 1 shows a schematic overview of a system according to the present disclosure. The system comprises a oxygen supply 1, for example in the form of a liquid oxygen tank (LOX), oxygen cylinders and/or a pressure swing for producing medical grade oxygen. The oxygen supply 1 supplies pure medical oxygen.

A supply for medical air 2 is present, wherein the medical air is typically produced by air compressors with a filtering and cleaning system which filters and cleans ambient air to produce medical grade air, or it is supplied by means of cylinders, bundles of cylinders or a cryogenic storage. The medical oxygen provided by the oxygen supply 1 as well as the medical air provided by the medical air supply 2 are supplied throughout a medical facility, in the current case a hospital, via a pipeline system. Medical oxygen is supplied by pipelines 10 and medical air is supplied by pipelines 20.

A gas blender 3 is present into which medical oxygen as well as medical air is fed by means of the respective pipelines 10, 20. The gas blender 3 is set for blending the medical oxygen and the medical air in a predetermined blending ratio, resulting in an oxygen content in the gas blend of between 30% volume to 96% volume. Accordingly, a desired predetermined oxygen content of the resulting gas blend can be achieved. The gas blend is supplied, within the hospital, via pipelines 30 and is made available to be used for treating a patient via a supply interface in the form of a wall outlet 32 in a patient room. Accordingly, a gas blend of medical oxygen and medical air is available in a patient room via the wall outlet 32.

From the wall outlet 32, the medical practitioner, the doctor and/or the nurse can take the gas blend by means of a suitably shaped plug or other means for connecting a device for treating a patient to the wall outlet 32 and may administer the gas blend via the device to the patient to be treated.

A device for treating a patient is understood to comprise any device which is intended to transport the gas blend from the wall outlet 32 to the patient in order to administer the gas blend to the patient. Using a device for treating the patient is indispensable in the present system because the wall outlet 32 is located spaced apart from the head of the patient. Accordingly, at least the provision of a tube would be necessary to transport the gas blend from the wall outlet to the patient in order to administer the gas blend to the patient. Typically such a device for treating a patient would be present in the form of a nasal cannula, a catheter, a mask or any other suitable device for transporting the gas blend from the wall outlet to a patient.

The gas blender 3 preferably can be set to control the gas blend to an oxygen content of the gas blend of between 30% by volume to 96% by volume, preferably between 40% by volume to 80% by volume, more preferred between 45% by volume and 70% by volume, most preferred of 50% by volume. In this respect, it is to be considered that the medical air which is received from the medical air supply 2 already comprises 21% of oxygen such that, in order to achieve the desired gas blending ratio, this oxygen content of 21% has to be topped-up by the medical oxygen received from the oxygen supply 1 accordingly.

The medical oxygen supply 1 as well as the medical air supply 2 are preferably situated in a centralized area I of a medical facility, in particular of a hospital, and are intended to supply the entire medical facility, or at least a unit of the medical facility, with medical oxygen and medical air.

The gas blender 3 is preferably situated at the ward gas entry of a hospital ward or at any specified hospital unit II. Accordingly, a number of gas blenders 3 could be present in a hospital, wherein the respective gas blenders 3 are intended to supply the respective hospital units or hospital wards with either identical gas blending ratios or with different gas blending ratios, depending on the structure of the conditions of the patients treated in the respective hospital area.

The wall outlet 32 for supplying the gas blend of medical oxygen and medical air to a device for treating a patient is situated in a patient room III such that the gas blend can be administered to a patient. Typically, in each and every patient room at least one set of gas wall outlets is present for supplying medical gases for treating the patient. Typically, an individual set of gas wall outlets is present per patient bed in a patient room.

In other words, the wall outlet 32 and the gas blender 3 are typically situated in a spaced apart fashion in a medical facility, and, in particular, in different rooms. The communication of the gases between the centralized area I, the respective ward entries or subunits of a hospital II, and the respective patient rooms III is provided by means of the hospital pipeline system, comprising at least the schematically depicted pipelines 10, 20 and 30. The pipeline system for the supply and distribution of medical gases is typically already present in a hospital.

The pipeline 30 which is used for supplying the gas blend of medical oxygen and medical air into the patient room III and, in particular, to the wall outlet 32 situated in this room, is already present in a hospital but was conventionally used for supplying pure medical oxygen to the patient room.

A set of gas wall outlets preferably comprises, besides the wall outlet 32 for the gas blend, another wall outlet 22 for supplying medical air from the medical air supply 2 to the patient room.

Typically, a vacuum supply 4 in the form of a vacuum pump is also present in a hospital, wherein the vacuum is transported from a centralized area I where the vacuum supply 4 is situated, via a pipeline system 40 to a patient room III, where it is supplied via yet another wall outlet 42.

The vacuum supply 4 is used for all suction applications in a patient room, such as to aspirate mucus from a patient. Medical air from the medical air supply 2 is used for all blowing and drying applications which might become necessary when treating a patient.

In a different embodiment, the gas blender 3 could also be situated in a centralized area I of the respective medical facility such as to supply the entire medical facility, or at least a large area of the medical facility, with the gas blend. The positioning of the gas blender 3 in the layout of the pipeline system depends on the actual requirements in the respective medical facilities. If it is required that the oxygen content of the gas blend of oxygen and medical air can be adjusted for specific areas in the respective medical facility, a number of gas blenders 3 are preferably provided at the respective unit entries or gas ward entries II. However, if a uniform blending ratio of oxygen and medical air is sufficient for the entire medical facility, the gas blender 3 could also be situated centrally. In addition, it very much depends on the layout of the pipelines already present in the hospital where a gas blender 3 could be situated. It is preferred to avoid excessive changes in the layout of already existing pipeline systems.

The gas blender 3, or at least one of the number of different gas blenders 3 of the system, preferably has an emergency bypass 34 which enables a user in an emergency to immediately switch to pure medical oxygen for the output of the gas blender 3 and/or the gas supplied to the pipeline 30, should this be required.

The provision of the gas blend of medical air and medical oxygen to a patient room III via wall outlet 32 leads to a reduction in the consumption of medical oxygen in units of the medical facility which do not need a pure medical oxygen supply. For example, even though acute care patients or patients in an operating room may need a pure medical oxygen supply, chronic or stable patients which are treated in long term hospital units may require lower oxygen contents such as, for example, a 60% medical oxygen concentration. By means of the supply of the gas blend of oxygen and medical air medical oxygen could be saved because the medical air already carries about 21% oxygen content, on the one hand side, and the waste of medical oxygen when administering the gas to the patient is reduced by the reduction in oxygen content of the gas blend.

When supplying the gas blend to a patient, thus, it will never occur that a gas blend is delivered to the patient which is potentially harmful because pure medical oxygen cannot be accidentally supplied to a patient anymore.

Furthermore, the gas blend supplied to the patient may be a pharmaceutical grade gas because the medical oxygen is not mixed with ambient air but rather with medical air.

In the system shown in Figure 1, a setup for intensive care units IV is given, wherein vacuum is provided at a wall outlet 42, medical air is provided at a wall outlet 22 but pure medical oxygen is supplied at a wall outlet 12.

Preferably, the gas wall outlets provided in a patient room III consist of a wall outlet for vacuum 42, a wall outlet for medical air 22 as well as a wall outlet for the gas blend of medical air and oxygen 32. The gas wall outlets for an emergency unit or an operating room IV preferably consist of a wall outlet for vacuum 42, a wall outlet for medical air 22 as well as a wall outlet for pure medical oxygen 12.

It is preferred to mark the wall outlet 32 for supplying the gas blend of oxygen and medical air as to its actual concentration of oxygen. It is also contemplated using different wall connectors which are coded mechanically with respect to the oxygen concentration actually supplied such that devices which are specifically designed for specific oxygen contents can only be connected to the correct wall outlet. In particular, it is contemplated using a different wall outlet socket for the gas blend of oxygen and medical air than the one that is typically used for pure medical oxygen.

In a further preferred embodiment, the gas blender communicates with a control system for controlling the gas blender as to the blending ratio. The control system and the gas blender are typically situated in a spaced apart manner, in particular such that the gas blender can be controlled remotely from the control system. The actual blending ratio may be verified by means of at least one sensor, for example an oxygen sensor, for sensing at least one components of the blended gas. This sensor preferably communicates with the remote system in order to control the blended gas and to log the concentrations of the gases in the blended gas fed into the hospital pipeline system and/or the ward pipeline system.

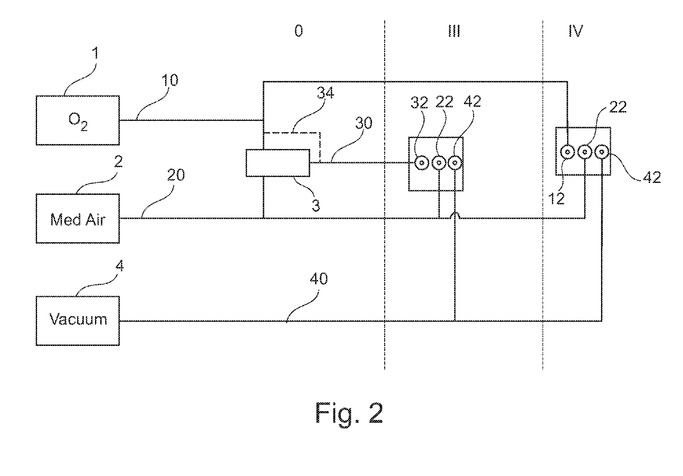

Figure 2 shows a further embodiment of the present disclosure wherein the general layout of the system resembles the system shown in Figure 1. However, the gas blender 3 is situated in an area O outside of the hospital, preferably in the vicinity of the tanks 1 and 2. By the provision of the gas blender 3 in the proximity of the respective gas supplies 1, 2 the amount of gas tubes to be installed is reduced since the two lines - oxygen and medical air - are reduced to one single line already in an early stage, namely reduced to the line carrying the blended gas. The blending process may also take place before the respective gas is shipped to the hospital.

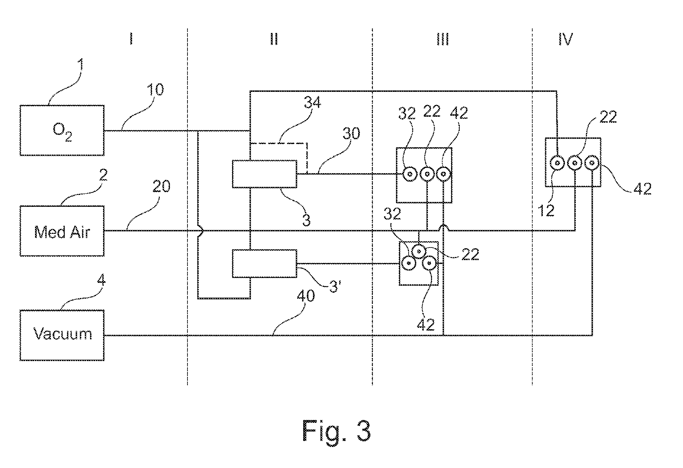

In Figure 3, a system similar to the system shown in Figure 1 is suggested but two gas blenders 3, 3' are provided. The provision of two gas blenders 3, 3' is intended to be exemplary for any number of gas blenders which could be used in the system. The gas blenders 3, 3' may be present at the entrance of a plurality of wards or at any other suitable location within the hospital. The presence of more than one gas blender 3 ensures that different blending ratios can be chosen dependent upon the needs of the respective wards or segments of the hospital. For example, a ward specializing in long-term patients may need a blending ratio such that a content of 60% oxygen is provided, whereas a different ward specializing in acute patients may require a blending ratio resulting in a content of 80% oxygen in the gas fed into the ward pipeline system. By the provision of more than one gas blender the system is even more flexible and the efficiency and safety can be even more improved by adapting the respective blending ratios individually to the needs of specific wards.

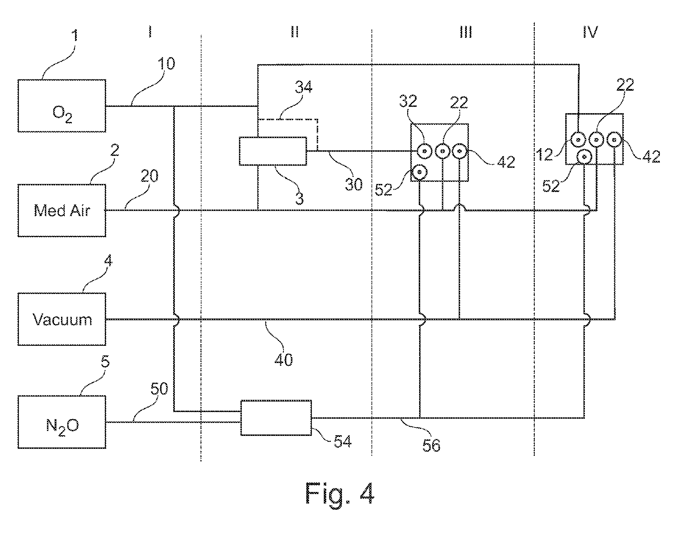

Figure 4 shows yet another embodiment which includes, in addition to the layout shown in and discussed with respect to Figure 1 to 3, a supply of nitrous oxide 5 which is fed into a pipeline 50. Nitrous oxide (N20) is used in medical applications for analgesic and anesthetic treatments. Another gas blender 54 is present for blending the nitrous oxide with the medical oxygen supplied by the medical oxygen supply 1. By means of the gas blender 54 a blended gas may be produced which may be directly administered to a patient. The blended gas is preferably fed into another pipeline 56 which supplies the blended gas to a wall outlet 52 in a patient room III and/or operating room IV such that the gas can be taken from the wall outlet 52 and directly applied to a patient for anesthetic and/or analgesic treatments. The advantage of this system is that the accidental administration of pure nitrous oxide, i.e. of an hypoxic gas, can be avoided. The blending ratio for nitrous oxide and oxygen is preferably adjustable such that a concentration of between 25% and 75% nitrous oxide are provided for in the blended gas, wherein the concentration is dependent on the intended application of the gas blend. For painkilling applications, a concentration of 50% nitrous oxide is preferred. For anesthetic applications, a concentration of 75% nitrous oxide is preferred. Accordingly, the gas blend may be supplied to operating rooms, delivery rooms, emergency rooms, intensive care units or any other room in which a patient is to be treated.

Preferably, other medical gases can be supplied in a similar fashion, namely by blending them with the medical oxygen already present in the system and by feeding the resulting blend into a pipeline system to make it available in a patient room by means of a wall outlet.

## Claims

1. System for supplying a medical gas in a medical facility, preferably in a hospital, the system comprising:
- at least one gas blender (3) for blending medical air and medical oxygen in a predetermined blending ratio; and
- at least one supply interface situated in a patient room (III), preferably a wall outlet (32), for supplying the gas blend to a device for treating a patient.

2. System according to claim 1, further comprising a supply of medical oxygen (1), a supply of medical air (2), and pipelines (10, 20) for connecting the supply of medical air (2) and the supply of medical oxygen (1) to the gas blender (3), and at least one pipeline (30) for connecting the gas blender (3) to the supply interface (32).

3. System according to claim 1 or 2, wherein the gas blender (3) is set to provide an oxygen content in the gas blend of between 30% by volume to 96% by volume, preferably between 40% by volume to 80% by volume, more preferred between 45% by volume to 70% by volume, most preferred of 50% by volume.

4. System according to any of the preceding claims, further comprising, in the patient room (III), a supply interface (22) for supplying medical air, and a supply interface (42) for supplying a vacuum.

5. System according to any of the preceding claims, wherein the gas blender (3) is situated at a ward gas entry (II) of a hospital ward, wherein the gas blend is preferably supplied from the ward entry to the ward at a pressure of between 2 and 6 bar gauge (barg), more preferred at between 3 and 5 bar gauge (barg), most preferred at about 4 bar gauge (barg).

6. System according to any of the preceding claims, wherein the gas blender (3) is situated in an area of the hospital (I, II) which is spaced apart from the patient room (III), preferably wherein the gas blender (3) is situated in a different room than the supply interface (32).

7. System according to any one of the preceding claims, wherein at least two gas blenders (3, 3') are provided which are set to supply blended gas with blending ratios distinct from one another, wherein preferably at least one gas blender (3, 3') is located at each ward gas entry (II) of at least two different wards in order to supply blended gases having blending ratios which are distinct from one another to the different wards.

8. System according to any one of claims 1 to 6, wherein a single gas blender (3) is provided, preferably in a centralized location or outside of the hospital, for supplying specific sections or the entire hospital with the gas blend in a predetermined gas blending ratio, wherein the gas blend is preferably supplied at a pressure of between 5 and 15 bar gauge (barg), more preferably between 7 and 13 bar gauge (barg), even more preferred between 8 and 11 bar gauge (barg), most preferred at 10 bar gauge (barg) to the sections or the entire hospital.

9. System according to any of the preceding claims, wherein the oxygen supply (1) comprises a liquid oxygen tank, oxygen bottles, oxygen cylinders and/or a pressure swing adsorption means for producing medical oxygen from ambient air.

10. System according to any of the preceding claims, wherein the medical air supply (2) comprises air compressors with filters and/or membranes for filtering ambient air to produce medical air and/or a centralized supply system including a tank for storing liquid medical air and/or cylinders containing medical air and/or cylinder bundles containing medical air.

11. System according to any of the preceding claims, wherein the supply interface (32) is a wall outlet and/or a socket for receiving a plug and/or a tube of a device for treating a patient, wherein the supply interface (32) is preferably marked and/or shaped individually to inform users of the blending ratio supplied via the respective supply interface.

12. System according to any of the preceding claims, wherein individual patient rooms (III) in the medical facility are supplied with an individual gas blend by individual gas blenders (3) in order to provide individual blending ratios to the individual patient rooms.

13. System according to any of the preceding claims, wherein the gas blender (3) comprises an emergency bypass (34) and/or an emergency valve to switch from supplying the gas blend to outputting pure medical oxygen.

14. System according to any of the preceding claims, further comprising a supply of nitrous oxide (5) and/or a supply of any other medical gas, and at least one further supply interface, preferably a wall outlet (52), in a patient room (III) for supplying the nitrous oxide and/or the medical gas to a device for treating a patient.

15. System according to claim 14, further including a blender (54) for blending the nitrous oxide and/or the medical gas with medical oxygen and/or with medical air in a predetermined blending ratio, wherein the gas blend is supplied to a device for treating a patient via the supply interface, preferably the wall outlet (52), in the patient room (III).

16. System according to any one of the preceding claims, further including a control system for remotely controlling the at least one gas blender (3, 3'), preferably including means for measuring the blending ratio in the blended gas flow and for communicating the measured blending ratio to the control system.

17. Method for supplying a medical gas in a medical facility, the method comprising the steps:
- blending medical air and medical oxygen in a predetermined blending ratio by means of at least one gas blender (3); and
- supplying the gas blend to a device for treating a patient by means of at least one supply interface situated in a patient room (III), preferably a wall outlet (32).
